# EUROPÄISCHE PATENTSCHRIFT

(11) **EP 2 310 841 B1**
(45) Veröffentlichungstag und Bekanntmachung des Hinweises auf die Patenterteilung: **10.04.2013**
(21) Anmeldenummer: 09780536.0
(22) Anmeldetag: 14.07.2009
(51) Int. Cl.: G01N 27/12

(54) **WIDERSTANDSGASSENSOR FÜR DIE BESTIMMUNG EINES REDUZIERBAREN GASES**
RESISTIVE GAS SENSOR FOR DETERMINING A REDUCIBLE GAS
CAPTEUR DE GAZ RÉSISTIF POUR DÉTERMINER UN GAZ RÉDUCTIBLE

(30) Priorität: 05.08.2008 DE 102008036370
(43) Veröffentlichungstag der Anmeldung: 20.04.2011
(73) Patentinhaber: Deutsches Zentrum für Luft- und Raumfahrt e.V., 51147 Köln (DE)
(72) Erfinder: SARUHAN-BRINGS, Bilge, 53859 Niederkassel (DE); STRANZENBACH, Mathias Christian, 10249 Berlin (DE); LEYENS, Christoph, 52639 Königswinter (DE)
(74) Vertreter: von Kreisler Selting Werner
(86) Internationale Anmeldenummer: PCT/EP2009/058954
(87) Internationale Veröffentlichungsnummer: WO 2010/015490

(56) Entgegenhaltungen:
- EP-A- 0 331 050
- DE-A1- 4 334 672
- JP-A- 63 066 448
- JP-A- 63 121 744

## Beschreibung

Die Erfindung betrifft eine Vorrichtung zur qualitativen und/oder quantitativen Bestimmung wenigstens einer Komponente eines chemisch reduzierbaren Gasgemisches, einen Abgaskatalysator, welcher sich einer solcher Vorrichtung bedient, ein Fahrzeug, das einen solchen Abgaskatalysator umfasst, ein Verfahren zur Herstellung einer solchen Vorrichtung, ein Verfahren zur Überwachung der NOₓ-Emissionen eines Fahrzeuges sowie die Verwendung einer solchen Vorrichtung.

Der Fahrzeugkatalysator, auch kurz *Katalysator* (umgangssprachlich *Kat),* dient der Abgasnachbehandlung in Fahrzeugen mit Verbrennungsmotor. Durch den Katalysator können die Schadstoffemissionen im Abgas drastisch reduziert werden. Im Allgemeinen wird die gesamte Anlage zur Abgasnachbehandlung als Fahrzeugkatalysator bezeichnet.

Der Fahrzeugkatalysator besteht in der Regel aus mehreren Komponenten. Als Träger dient ein temperaturstabiler Wabenkörper aus Keramik (Monolith) oder Metallfolien (Metalit), der eine Vielzahl dünnwandiger Kanäle aufweist. Auf dem Träger befindet sich der sogenannte Washcoat. Er besteht aus sehr porösem Aluminiumoxid (Al₂O₃) sowie aus Sauerstoffspeicherkomponenten wie zum Beispiel Ceroxid und dient zur Vergrößerung der Oberfläche. Durch die hohe Oberflächenrauhigkeit wird eine sehr große Oberfläche realisiert (bis zu mehreren tausend Quadratmetern). In einem Washcoat sind die katalytisch aktiven Edelmetalle eingelagert. Bei modernen Abgaskatalysatoren sind dies die Edelmetalle Platin, Rhodium und/oder Palladium. Der keramische Träger is mithilfe spezieller Matten (in der Regel Fasermatten, seltener Drahtgestricke) in einem metallischen Gehäuse, dem sogenannten Canning, gelagert. Das Canning der Metall-Katalysatoren reduziert sich auf das Einschweißen des MetallKatalysators in den Abgasstrang. Spezielle Matten oder ein extra Metallgehäuse sind nicht notwendig. Das Canning ist fest im Abgasstrang des Fahrzeugs verbaut und besitzt zum Teil weitere Anschlussmöglichkeiten für zum Beispiel Lambdasonden oder Thermoelemente. Es gibt auch Metall-Katalysatoren mit integrierten Lambdasonden.

Die Wirkungsweise von Fahrzeugkatalysatoren beruht auf katalytischen Reaktionen. Die Aufgabe des Fahrzeugkatalysators ist die chemische Konvertierung der Verbrennungsschadstoffe Kohlenwasserstoffe (HₘCₙ), Kohlenstoffmonoxid (CO) und Stickoxide (NOₓ) in die ungiftigen Stoffe Kohlenstoffdioxid (CO₂), Wasser (H₂O) und Stickstoff (N₂) durch Oxidation beziehungsweise Reduktion. Je nach Betriebspunkt des Motors und können bei optimalen Betriebsbedingungen Konvertierungsraten nahe 100 % erreicht werden.

Für die vorliegende Erfindung von Bedeutung sind insbesondere die folgenden drei verschiedenen Katalysator-Typen:

### 1. Drei-Wege-Katalysator:

Bei einem Drei-Wege-Katalysator (auch G-Kat genannt) finden die Oxidation von CO und HₘCₙ sowie die Reduktion von NOₓ parallel zueinander statt: Es werden HₘCₙ mit O₂ zu CO₂ und H₂O oxidiert, CO mit O₂ zu CO₂ oxidiert und NOₓ mit CO zu N₂ und CO₂ reduziert.

Voraussetzung dafür ist ein konstantes Luft-Kraftstoff-Gemisch im stöchiometrischen Verhältnis (λ = 1) von 14,7 g Luft pro g Benzin-Kraftstoff. Schon eine geringe Abweichung in den mageren Bereich (λ > 1) bewirkt einen sprunghaften Anstieg der Stickoxidemission hinter dem Katalysator, da zu wenig CO für die Reduktion vorhanden ist. Deshalb wird das Gemisch zwischen stöchiometrischem und leicht fettem Verhältnis geregelt. Der Drei-Wege-Katalysator kann nur bei Fahrzeugen mit Ottomotor und Lambdaregelung eingesetzt werden. Bei einem Dieselmotor verhindert der Sauerstoffüberschuss im Abgas die Reduktion des NOₓ und macht spezielle Katalysatoren erforderlich.

### 2. NOₓ-Speicherkatalysator:

Moderne Magermixmotoren arbeiten in einem Sauerstoffüberschuss zur Erhöhung des Motorwirkungsgrades. Herkömmliche Katalysatoren können daher nicht eingesetzt werden. Die Oxidation von CO und HₘCₙ ist im Sauerstoffüberschuss (λ > 1) analog zum herkömmlichen Dreiwegekatalysator weiterhin möglich, jedoch müssen Stickoxide (NOₓ) zwischengespeichert werden. Deren katalytische Reduktion gelingt nur in einem stöchiometrischen bis fetten Abgasgemisch. Diese neuen Motoren benötigen daher eine weiterentwickelte Art von Katalysatoren mit zusätzlichen chemischen Elementen, die eine Speicherung von Stickoxiden ermöglichen.

Ist die Aufnahmekapazität des Katalysators erschöpft, so wird seitens der Motorelektronik kurzzeitig ein fettes, reduzierendes Abgasgemisch eingestellt (circa zwei Sekunden). In diesem kurzen, fetten Zyklus werden die im Katalysator zwischengespeicherten Stickoxide zu Stickstoff reduziert und damit der Katalysator für den nächsten Speicherzyklus vorbereitet. Durch dieses Vorgehen ist es auch möglich, die Schadstoffemissionen sparsamer Magermixmotoren zu minimieren und gültige Grenzwerte der Euro-Normen einzuhalten. Die Aufnahmekapazität (circa 60 bis 90 Sekunden) wird durch einen NOₓ-Sensor überwacht.

Um diese Zwischenspeicherung der Stickstoffoxide zu erreichen, werden auf geeigneten Trägern ein Edelmetallkatalysator wie Platin und eine NOₓ-Speicherkomponente, die meistens ein Erdalkalimetall wie Barium ist, aufgebracht. In der mageren, das heißt sauerstoffreichen, Atmosphäre werden die Stickstoffoxide unter der katalytischen Wirkung des Edelmetallkatalysators aufoxidiert, unter Ausbildung von Nitraten wie beispielsweise Bariumnitrat im Katalysator absorbiert und somit aus dem Abgasstrom entfernt. Durch das regelmäßige kurzzeitige "Anfetten" laufen diese Reaktionen in der entgegengesetzten Richtung ab, wodurch die NOₓ wieder in den Abgasstrom abgegeben und durch die in der fetten Atmosphäre vorhandenen, reduzierenden Komponenten wie HₘCₙ - unvollständig verbrannte Kohlenwasserstoffe - oder CO weiter reduziert werden. Der Speicherkat kann NOₓ nur in einem Temperaturbereich von 250 bis 500 Grad Celsius speichern. Das Temperaturfenster wird durch dreiflutige Abgasrohre oder Auspuffbypässe erreicht.

### 3. SCR (Selective Catalytic Reduction, Selektive Katalytische Reduktion):

Ein weiteres, angestrebtes und mittlerweile marktreifes Verfahren zur Reduktion von Stickoxiden ist die selektive katalytische Reduktion. Hierbei wird kontinuierlich eine wässrige Harnstofflösung (Handelsname AdBlue) in den Abgasstrom eingespritzt, aus welcher durch Hydrolyse Wasser und Ammoniak entstehen. Das somit entstandene Ammoniak ist in der Lage, die Stickoxide im Abgas zu Stickstoff zu reduzieren.

Die Überwachung der Zusammensetzung des Abgasstromes eines Fahrzeugkatalysators erfolgt heutzutage standardmäßig mit Hilfe der sogenannten Lambdasonde.

Die Messung basiert auf dem Restsauerstoffgehalt im Abgas. Sie ist der Hauptsensor im Regelkreis der Lambdaregelung zur katalytischen Abgasreinigung (umgangssprachlich: geregelter Katalysator). Es werden zwei Messprinzipien verwendet: Spannung eines Festkörperelektrolyten (Nernstsonde) und Widerstandsänderung der Keramik (Widerstandssonde).

Von diesen beiden Sondenarten ist die Nernstsonde die weit aus gebräuchlichere und soll daher im Folgenden kurz dargestellt werden.

Bei der Nernstsonde ist eine Seite des keramischen Sensorelements dem Abgasstrom ausgesetzt, während die andere Seite an einer Sauerstoffreferenz liegt. In der Regel wird hierzu die Umgebungsluft verwendet, entweder durch eine Öffnung direkt an der Sonde oder über die Zuleitung und den Stecker. Dies erschwert die sogenannte Referenzluftvergiftung durch Wasser, Öl- oder Kraftstoffdämpfe. Dabei ist der Sauerstoffgehalt der Referenz verringert und die Sondenspannung verkleinert. Bei einer gepumpten Referenz wird die Umgebungsluft nicht mehr benötigt, sondern die Sauerstoffreferenz wird durch einen aufgeprägten Sauerstoffionenstrom aus dem Abgas hergestellt.

Bei Temperaturen über etwa 300 °C wird die Yttrium-dotierte Zirkoniumdioxid-Keramik der Sonde für negative Sauerstoff-Ionen leitend. Der Konzentrationsunterschied erzeugt eine Ionendiffusion zum Abgas. Die Sauerstoff-Atome können als doppelt negativ geladene Ionen durch die Keramik durchtreten. Die zur Ionisierung der Sauerstoff-Atome erforderlichen Elektronen werden von den elektronisch leitfähigen Elektroden geliefert. Dadurch lässt sich zwischen den innen und außen angebrachten Platin-Elektroden eine elektrische Spannung abnehmen, die *Sondenspannung.* Diese wird über die Verkabelung an das Motorsteuergerät weitergeleitet. Sie liegt bei λ > 1 (mageres Gemisch, zuviel Luft) zwischen 0 und 150 mV, bei λ < 1 (fettes Gemisch, zuviel Kraftstoff) zwischen 800 und 1000 mV. Die Spannung wird dabei durch die Nernst-Gleichung beschrieben. In einem sehr schmalen Übergangsbereich um λ = 1, dem sogenannten λ-Fenster, ist die Kennlinie extrem steil. Die Spannung ändert sich dort in Abhängigkeit vom Luft-Kraftstoff-Verhältnis fast sprunghaft.

Die Sonde wird bei Ottomotoren in der Regel in den Abgaskrümmer oder kurz hinter das Sammelrohr montiert. In Fahrzeugen mit hohen gesetzlichen Anforderungen an die Abgasreinigung und die Eigendiagnose kommen mehrere Sonden zum Einsatz, bei V-Motoren in der Regel pro Zylinderbank, bis zu einer Sonde pro Zylinder für eine selektive Zylinderregelung.

Das korrekte Lambdaverhältnis ist ein wichtiger Parameter zur Steuerung der Verbrennung und zu Ermöglichung der Abgasreinigung durch den Drei-Wege-Katalysator. Im Fahrzeugbereich hat sich die Lambdasonde aufgrund der gesetzlichen Einschränkung der Abgasemissionen zuerst in den USA und nachfolgend auch in Europa durchgesetzt.

Die ersten Lambdasonden wurden als "Fingersonden" gebaut. Das eigentliche Sensorelement ist dabei wie ein Hütchen geformt, mit dem Abgas außen und der Referenzluft im Inneren.

Zunehmend werden die Sensoren in Planartechnik aus mehreren Schichten aufgebaut bei denen auch die Sondenheizung bereits integriert ist.

Das keramische Element ist von einem sogenannten Schutzrohr umgeben. Es erleichtert, das Sensorelement auf der gewünschten Temperatur zu halten und beugt mechanischen Schäden vor. Für den Gaszutritt ist das Schutzrohr mit Löchern versehen.

Die Lambdasonde vergleicht permanent den Restsauerstoffgehalt im Abgas mit dem Luftsauerstoffgehalt und leitet diesen Wert als analoges elektrisches Signal an ein Steuergerät, das zusammen mit anderen Kenngrößen daraus ein Steuersignal zur Gemischbildung erzeugt, was im Ottomotor im Allgemeinen in der Anpassung der Einspritzmenge mündet. Bei OBD-Fahrzeugen muss die Funktion der Regel-Lambdasonde und Monitorsonde vom Steuergerät überwacht werden. Die Überwachung erfolgt sporadisch.

Ein bedeutender Nachteil bei der Verwendung von Lambdasonden ergibt sich im Allgemeinen aus den relativ hohen Kosten, welche diese Sonden verursachen. Hinzu kommt, dass sie aufwendig in den Katalysator bzw. an Stellen vor und/oder hinter dem Katalysator integriert werden müssen. Auch ist durch diese Lambdasonden keine direkte Funktionsüberwachung des Katalysators möglich.

Einen ersten Ausweg aus dieser fehlenden direkten Überwachung scheinen Temperatursensoren aufzuzeigen, wie sie zum Beispiel in US 5,431,012, EP 1 389 268 B1 und EP 0 769 096 B1 beschrieben sind. Eine Überwachung der Abgaszusammensetzung gelingt über solche Temperatursensor-integrierte Abgaskatalysatoren jedoch nicht. An dieser Stelle wären Gassensor-integrierte Abgaskatalysatoren wünschenswert, welche bis dato jedoch nicht beschrieben sind.

Die Kombination eines Katalysators mit einem Gassensor ist zum Beispiel in "An integrated solution for NOₓ-reduction and -control under lean-burn conditions" (B. Saruhan, M. Stranzenbach, G.C. Mondragön-Rodriguez, Mat.-wiss. U. Werkstofftech. 2007, 38, 725 bis 733) beschrieben. Eine solche Kombination eignet sich jedoch nicht zur Verwendung in einem Abgaskatalysator, da der eigentliche Katalysator und der Gassensor räumlich voneinander getrennt vorliegen.

JP 631 21 744, DE 43 34 672 und JP 3 60 66 448 offenbaren Gassesoren mit Katalytischen oxiations-oder Redulationskonverter schichten.

Es ist also eine Aufgabe der vorliegenden Erfindung, eine Gassensor-integrierte Vorrichtung zur Verfügung zu stellen, welche sich zum Beispiel in einem Abgaskatalysator einsetzen lässt, die eine direkte Funktionsüberwachung des Katalysators gestattet und sich zudem kostengünstig realisieren lässt.

Darüber hinaus ist es eine weitere Aufgabe der vorliegenden Erfindung, die Integration des Gassensors so gestalten zu können, dass sich dieser zur Alterungsbestimmung des Katalysators heranziehen lässt.

Die der vorliegenden Erfindung zugrundeliegende Aufgabe wird gemäss Ansprüche 1, 8, 11 und 12 gelöst.

Die Reduktionsschicht, die Oxidationsschicht und die selektive Gassensor-Elektrode sind dabei zunächst als räumlich voneinander unabhängige Entitäten aufzufassen, das heißt insbesondere, dass diese Schichten bzw. Elektroden nicht notwendigerweise in Kontakt stehen. Die räumliche Unabhängigkeit dieser mindestens drei Bestandteile der Vorrichtung lässt sich jedoch nur soweit realisieren, als sie in einem Bauteil integriert sind, was die Anordnung dieser erfindungsgemäßen Vorrichtung eindeutig von der in der oben schon angesprochenen Publikation "An integrated solution for NOₓ-reduction and -control under lean-burn conditions" abgrenzt (wobei letztere weiterhin auch keine Oxidationsschicht aufweist).

Unter (chemischer) Reduktion bzw. Oxidation ist in diesem Zusammenhang nicht notwendigerweise nur der Verlust bzw. die Aufnahme von Sauerstoff zu verstehen. Vielmehr sind die Begriffe "Reduktion" bzw. "Oxidation" im vorliegenden Fall soweit gefasst, dass sie sich auf eine Erniedrigung bzw. eine Erhöhung der Oxidationszahlen der jeweils betrachteten Elemente beziehen. Auch der Begriff der "Rückoxidation" ist nicht einschränkend zu verstehen, dergestalt, dass eine einmal zu einer Verbindung C reduzierte Ausgangsverbindung A nicht notwendigerweise wieder zu A zurückoxidiert werden muss. Vielmehr ist auch eine Reduktion zu einer Verbindung B mit einer mittleren Oxidationszahl, welche zwischen den Oxidationszahlen der Verbindungen A und C liegt, möglich.

Bevorzugt ist/sind die Gassensor-Elektrode oder/und die Reduktionsschicht oder/und die Oxidationsschicht mit einem Trägersubstrat verbunden. Dieses Trägersubstrat verleiht der erfindungsgemäßen Vorrichtung zusätzlich Stabilität und ermöglicht eine nahezu beliebige Anordnung der mindestens drei Bestandteile der Vorrichtung zueinander.

Bevorzugt handelt es sich bei dem Trägersubstrat um einen keramischen Wabenkörper oder eine Metallfolie. Zu beachten ist, dass in den Fällen, in denen das Trägersubstrat elektrisch leitend ist, wie zum Beispiel bei einer Metallfolie, das Trägersubstrat von den gegebenenfalls vorhandenen metallischen Katalysatorpartikeln in der Reduktions- und/oder Oxidationsschicht elektrisch isoliert vorliegt. Dies kann zum Beispiel durch ein zusätzliches Aufbringen einer katalytisch inerten Schicht (zum Beispiel Al₂O₃) erreicht werden.

Die mindestens drei Bestandteile der Vorrichtung, das heißt die Reduktionsschicht die Oxidationsschicht sowie die selektive Gassensor-Elektrode sind so angeordnet, dass die zu reduzierende/oxidierende Komponente auf ihrem Weg aus dem Gasgemisch zur Gassensor-Elektrode zunächst die Reduktionsschicht und dann die Oxidationsschicht passiert. Durch einen solchen Aufbau lässt sich eine genaue und reproduzierbare Detektion der rückoxidierten Komponente durch die dafür selektive Gassensor-Elektrode erreichen.

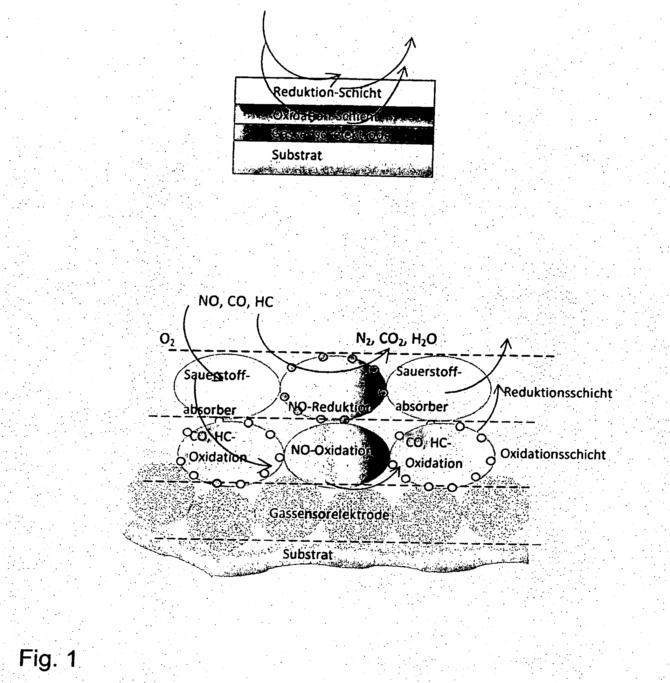

Anschaulich kann dieser Sachverhalt durch eine gedankliche Betrachtung der Detektion von NO₂ in einem Abgasstrom dargestellt werden (vgl. Fig. 1): Nach der Verbrennung enthält der resultierende Abgasstrom neben den eigentlichen Verbrennungsprodukten CO₂ und H₂O die weiteren unerwünschten Verbrennungsprodukte wie NOₓ, CO sowie UHC (unreacted hydrocarbons). Wie eingangs beschrieben, ist es die Aufgabe eines wie auch immer gearteten Abgaskatalysators, die drei letztgenannten Komponenten zu N₂, CO₂ und H₂O zu konvertieren. Treffen diese drei Komponenten nun zunächst auf die Reduktionsschicht, so werden diese unter ausreichend reduzierenden Bedingungen vollständig zu N₂, CO₂ und H₂O umgesetzt. Im Falle zu magerer Reaktionsbedingungen bleibt ein Teil dieser Komponenten jedoch unumgesetzt, insbesondere werden nicht alle NOₓ vollständig zu N₂ reduziert. Treffen diese unumgesetzt gebliebenen NOₓ auf die Oxidationsschicht, so werden diese zu NO₂ oxidiert und anschließend über die NO₂-selektive Gassensor-Elektrode detektiert. Mithin erfasst die NO₂-selektive Gassensor-Elektrode nur dann eine von 0 verschiedene NO₂-Konzentration, wenn das Kraftstoff-Luft-Verhältnis nicht optimal eingestellt ist.

Es ist nun leicht einzusehen, dass bei unabhängig voneinander angeordneten Reduktions- bzw. Oxidationsschichten eine solche selektive Detektion wesentlich schwieriger zu erreichen ist.

Weiterhin ist bevorzugt, dass die Gassensor-Elektrode eine Dicke in einem Bereich von 1 bis 5 µm aufweist.

Geringere Schichtdicken sind insofern benachteiligt, als eine zufriedenstellende Funktion der Gassensor-Elektrode nicht mehr gewährleistet ist/werden kann. Größere Schichtdicken sind von Nachteil, da sie mit einem höheren Verfahrensaufwand bei der Herstellung und vor allen Dingen höheren Kosten verbunden sind.

Die Gassensor-Elektrode ist ein einfaches, ein binäres, ein dotiertes Übergangsmetalloxid (z.B. TiO₂) oder ein Halbleiteroxid (z.B. SnO₂) Die Dotierungselemente für TiO₂ sind Fe, Zn, Zn/Al, Cu, Pt, Al, Nb und für SnO₂ Cu, Al, Au und W mit jeweils 1-6 at.%.

Die Reduktions- und die Oxidationsschicht sind bevorzugt gasdurchlässige Schichten mindestens eines, komplexen Oxids mit einer Spinell-, Perovskit- oder Aluminat-Struktur und umfassen darüber hinaus mindestens ein Edelmetall. Bei dem Edelmetall der Reduktionsschicht handelt es sich bevorzugt um Pd, Rh bei dem Edelmetall der Oxidationsschicht bevorzugt um Pt.

Die im Gitter eines Komplexoxides eingebundenen Edelmetalle reduzieren Schadgasse viel effektiver, benötigen einen mindesten 3mal geringeren Edelmetallanteil und sind stabiler gegen thermische Alterung (Edelmetallvergröberung bzw. Sinterung wird vermieden durch Ein- und Ausbau des Edelmetalls in das Gitter des Komplexoxids).

Außerdem soll im Falle eines metallischen Trägersubstrats eine elektrisch isolierende Schicht auf diesem verwendet werden.

Die Trägermaterialien umfassen bevorzugt hochporöse, insbesondere keramische Strukturen auf Siliziumoxid-, Ceroxid- und/oder Aluminiumoxid-Basis, wie sie zum Beispiel über im Stand der Technik hinlänglich bekannte Sol-Gel-Verfahren zugänglich sind einschließlich Cordierit, Mullit und/oder Aluminiumtitanat.

Die Reduktionsschicht weist bevorzugt eine Dicke in einem Bereich von 10 bis 20 µm auf, die Oxidationsschicht bevorzugt eine Dicke in einem Bereich von 1 bis 10 µm.

Die Reduktionsschicht muss eine Mindestschichtdicke aufweisen, um die im Abgas enthaltenen unerwünschten Bestandteile in zufriedenstellender Menge reduzieren zu können. Größere Schichtdicken sind entsprechend wieder mit höheren Kosten verbunden, wobei insbesondere das/die im Trägermetall enthaltenden Edelmetall/e ins Gewicht fallen.

Analoge Argumente hinsichtlich bevorzugter Schichtdicken treffen auch auf die Oxidationsschicht zu (das heißt nicht ausreichende Oxidation von NOₓ zu NO₂ bei zu geringer Schichtdicke; zu hohe Kosten bei zu großer Schichtdicke).

Weiterhin ist es bevorzugt, dass mindestens zwei Gassensor-Elektroden in Bezug auf eine Strömungsrichtung des Gasgemisches räumlich hintereinander angeordnet sind. Dieses Design bietet den Vorteil, dass die Detektion über die gesamte Länge des Katalysators erfolgen kann. So können bei einer solchen Segmentierung des Katalysators Informationen über den Alterungszustand des Katalysators gewonnen werden, da die katalytische Aktivität mit der Alterung im Katalysator nach hinten wandert und daher die Sensoren in Strömungsrichtung des Gasgemisches unterschiedliche NOₓ-Werte anzeigen können. Durch diese Art der Anordnung kann darüber hinaus auch die durch die Fahrdynamik hervorgerufene Temperatur-Abhängigkeit der Widerstandssensoren behoben werden. Die Schwankungen können dann durch vergleichende Messungen zwischen zwei Sensorelementen kompensiert werden. Das Sensorsignal entsteht also durch die Widerstandsdifferenz bzw. den -quotienten zwischen den einzelnen Signalen.

Eine Ausführungsform umfasst einen Abgaskatalysator, insbesondere einen Drei-Wege-Katalysator, einen Selective Catalytic Reduction(SCR)-Katalysator oder einen NOₓ-Speicherkatalysator, welcher eine erfindungsgemäße Vorrichtung gemäß Ansprüche 1-7 umfasst.

Solche Katalysator/Sensor-Schichten können in allen Verbrennungsanwendungen eingesetzt werden: andere Beispiele wären: Niederdruckturbine eines Flugzeuges, einer Stationären Turbine, Verbrennungs- und Heizungsanlagen, Wasserstofferzeuger und - Reinigeranlagen, etc.

Eine Ausführungsform umfasst ein Fahrzeug, welches einen erfindungsgemäßen Abgaskatalysator eingebaut enthält.

In einer Ausführungsform wird die der Erfindung zugrundeliegende Aufgabe gelöst durch ein Verfahren zur Herstellung einer erfindungsgemäßen Vorrichtung gemäss Ansprüche 1 bis 7. Im Fall eines metallischen Trägersubstrates befindet sich zwischen diesem und der Elektrode eine elektrisch nichtleitende Schicht (z.B. Al₂O₃).

Die Gassensor-Elektrode wird dabei bevorzugt durch CVD-, PVD-, Sol-Gel-, Plasmaspritz-, Siebdruck-, PLD-, Plattier- oder Tauchverfahren erzeugt.

Die Oxidations- und/oder Reduktionsschicht, insbesondere das Trägermaterial besagter Schichten, wird bevorzugt durch ein Sol-/Gel-Verfahren herstellt. Dies kann zum Beispiel dadurch geschehen, dass man eine Sol-Vorstufe des Trägermaterials mit einem Vorläufer des Edelmetall-Katalysators versieht und das Trägersubstrat mit einem solchen Sol und/oder entsprechend daraus hergestelltem Gel imprägniert. Nach Trocknung wird der Washcoat erhalten, in dem die Edelmetall-Katalysatorpartikel aus ihren Vorstufen nach einer kurzen Inbetriebnahme durch Reduktion gebildet werden.

Über ein Sol-Gel-Verfahren hergestellte edelmetallintegrierte Perowskite (oder Spinelle oder Aluminate) können auch über Pulvervorstufen im Tauchverfahren nach Zumischung eines bindenden Oxides (z.B. Siliziumoxid) auf dem Trägersubstrat aufgebracht werden.

In einer Ausführungsform wird die der Erfindung zugrundeliegende Aufgabe gelöst durch ein Verfahren zur Überwachung der NOₓ-Emissionen eines Fahrzeugs, welches dadurch gekennzeichnet ist, dass das Luft-Treibstoffverhältnis in Abhängigkeit von der/den Menge/n der Komponente/n variiert wird, für die die Gassensor-Elektrode selektiv ist.

In einer Ausführungsform wird die der Erfindung zugrundeliegende Aufgabe gelöst durch die Verwendung einer erfindungsgemäßen Vorrichtung zur qualitativen und/oder quantitativen Bestimmung wenigstens einer Komponente eines chemisch reduzierbaren Gasgemisches.

Insbesondere handelt es sich bei dieser Komponente um eine NOₓ-Verbindung, insbesondere um NO₂. Andere Emission und Gassorten (z.B. CO₂, H₂, UKW-unverbannte Kohlenwasserstoffe) sind ebenfalls durch Verwendung entsprechender Sensor-/Katalysatorschicht zu einschließen.

Weiterhin ist es bevorzugt, dass es sich bei dem Gasgemisch um das Abgas eines Verbrennungsmotors handelt.
Fig. 1 zeigt ein Beispiel einer erfindungsgemäßen Vorrichtung und ihre Verwendung innerhalb eines Abgaskatalysators (jeweils ausschnittsweise).

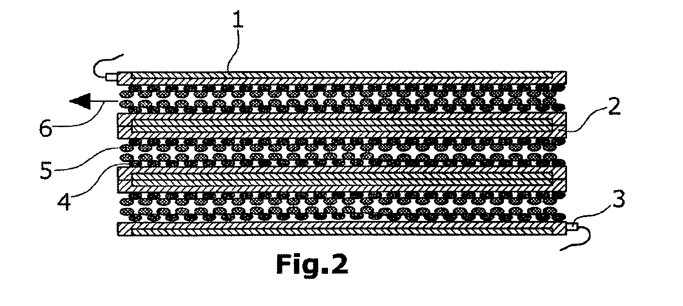

Fig. 2 zeigt detaillierter den eigentlichen Aufbau des Abgaskatalysators: Auf ein Trägersubstrat (1) aufgebracht ist dabei zunächst eine bzw. mehrere Gassensor-Elektrode/n (2), welche über die Kontaktierung/en (3) mit einer Detektierungseinheit (nicht dargestellt) verbunden sind. An die Gassensor-Elektrode (2) schließen sich in Richtung des Innenraumes des Trägersubstrats (1) die Oxidationsschicht/en (4) und die Reduktionsschicht/en (5) an. Durch einen solchen Aufbau wird gewährleistet, dass die Komponenten des Abgasstroms (6) zunächst die Reduktionsschicht/en (5) und anschließend die Oxidationsschicht/en (4) passieren müssen, bevor die gegebenenfalls chemisch veränderten Komponenten auf die Gassensor-Elektrode (2) treffen können.

Zusammenfassend wird durch die beschriebene Erfindung eine allgemeine, das heißt übergeordnete, erfinderische Idee verwirklicht, dass nämlich das gesamte System mindestens zwei Funktionen ausführt und sich mindestens ein Werkstoff dieses Systems mit seinen Eigenschaften an mindestens zwei Funktionen beteiligt. Darüber hinaus können diese Funktionen des Werkstoffsystems, beeinflusst durch externe Anregungen (mechanischer, chemischer oder physikalischer Natur), miteinander durch interne oder externe Steuerung interagieren.

### Ausführungsbeispiel:

### Ausführungsbeispiel I

Eine 0,5 mm Dicke Al₂O₃-Platte wurde erstmalig mit 4 at.% Cu-dotiertem TiO₂ aus zwei Quellen über Magnetron-Sputter-Verfahren reaktiv beschichtet. Typischerweise ergibt eine Beschichtungsdauer von 1-2 Stunden eine Schickdicke von 3-5 µm. Die beschichtete Platte wurden nachfolgend zur Kristallisation und zum Einbau einer porösen Morphologie bei 900 °C geglüht.

Zur Herstellung der Katalysatorreduktions- und Katalysatoroxidationsschichten wurden auf der Citrat- und Kofällungsmethode basierende Sols verwendet.

Die Katalysatoroxidationsschicht mit BaTi(_{1-z})Pt_{z}O₃ wurde über Kofällung von Ba- und Pt-Nitrat-Lösungen durch Zugabe von Ti-Lösung erreicht. Die Barium-basierte Lösung wurde durch Auflösen von metallischen Bariumstücken in Äthanol unter kontinuierlichem Rühren über Nacht präpariert. Dehydratisiertes Pt-Nitrat wurde zur Ba-Lösung addiert. Die Ti-Lösung wurde durch Auflösen von Titan-Isopropoxid (99.99%) in 2-Propanol vorbereitet. Die Kofällung wurde durch schnelle Zumischung der Ti-Lösung in die Ba-/Pt-Lösung erreicht. Nach dem Erhalten eines homogenen Sols wurden die Lösungsmittel bei 80 °C am Rotationsverdampfer entfernt. Das erhaltene Gel wurde bei Temperaturen bis 900 °C calciniert und einschließend 60 Minuten gemahlen.

Die Katalysatorreduktionsschicht mit der Zusammensetzung LaFeₓCo(_{1-x-y)}Pd_{y}O₃ wurde aus einem Citratsol, das aus drei wässrigen Lösungen entsteht (La-Acetat, Co-Acetylacetonat und Fe-Nitrat), präpariert. Zusätzlich wurde eine wässrige Pd-Nitrat-Lösung in 30 vol.% HNO₃ + Wasser in gewünschter Proportion (y) vorbereitet und zur Fe-Nitrat-Lösung zugemischt. Die Pd-/Fe-Nitrat-Lösung wurde unter Zugabe einer stöchimetrischen Menge von Zitronensäure in die Co-Lösung eingeführt. Die Zitronensäure bildet dabei Komplexe mit Metall-Kationen. Die resultierende Lösung wurde unter kontinuierlichem Rühren und Zugabe von Äthylenglykol (in 40:60 Massenverhältnis zu Zitronensäure) mit der La-Lösung gemischt. Das Lösungsmittel des Sols wurde bei 80 °C am Rotationsverdampfer entfernt. Dies führte zur Bildung eines schaumigen Fettstoffes, der leicht mit einem Mörser zerkleinert, bei Temperaturen von 500-900 °C für 3 Stunden calciniert und anschließend 60 Minuten gemahlen wurde.

Das entstehende feinkörnige Pulver wurde in das Citrat- oder Kofällungssol in einem Festkörper: Flüssigkeits-Verhältnis von 10:90 eingegeben. Die mit dotiertem TiO₂ vorbeschichtete Platte wurde zunächst mit der Katalysatoroxidations- und dann mit der Katalysatorreduktionsschicht beschichtet. Hierfür wurde die mit der Sensorschicht beschichtete Platte in die Sol+Pulver-Mischung eingetaucht, ein paar Minuten darin belassen und anschließend herausgezogen. Der Trocknungsprozess erfolgte in drei Schritten: zunächstwurden die beschichtete Platten für 1-2 Stunden an der Luft und anschließendim Trockenschrank bei 80 °C mindestens 3 Stunden getrocknet. Diese Prozedur wurde dreimal wiederholt. So entstehende Beschichtungen wurden anschließend bei 900 °C für 3 Stunden geglüht.

### Ausführungsbeispiel II

Zur Präparation von Cu-dotiertem Ti-Sol wurde Titan-Isopropoxid in 2-Propanol gelöst und mit einer wässrigen Cu-Nitrat-Lösung hydrolisiert. Eine Kordiarit (Al-Mg-Spinell) Wabenstruktur wurde in dieses kolloidale Sol eingetaucht und herausgezogen. Das überflüssige Sol wurde durch Druckluft entfernt. Die beschichte Wabenstruktur wurde zunächst an Luft, dann im Trockenschrank bei 150 °C getrocknet und anschließend bei 900 °C geglüht.

Mit den über Sol-Gel-Verfahren hergestellten und gemahlenen BaTi(_{1-z)}Pt_{z}O₃- (siehe oben für Katalysatoroxidationsmaterial) oder LaFeₓCo(_{1-x-y})Pd_{y}O₃- (siehe oben für Katalysatorreduktionsmaterial) Pulvern wurde eine wässrige Suspension mit einem 40:60 Feststoff:Wasser-Verhältnis vorbereitet. Die TiO₂-beschichtete Platte wurde in diese Suspension eingetaucht, herausgezogen und die überflüssige Lösung mit Druckluft entfernt. Dieser Schritt wurde dreimal wiederholt. Nach einem langsamen Trocknungsprozess bei 150 °C, wurden die Beschichtungen bei 500-700 °C calciniert.

Die Zusammensetzung der Katalysatoroxidationsschichten kann zum Beispiel durch Pt-dotierte Hexaluminate (LaMnAl₁₁O₁₉) oder LaSrMnPtO₃-Perowskite ersetzt werden.

Bei den Katalysatorreduktionsmaterialien kann statt Pd auch Rh verwendet werden.

### Alterung bzw. Schädigungskontrolle des Katalysators

Über ein im System integrierten Widerstands-Gassensor soll eine Alterungs- und Schädigungs-bedingte Fehlfunktion des Katalysators erkannt und an das OBD-System des Fahrzeugs übermittelt werden. Für den oben beschriebenen Katalysatortyp liegt immer genau dann eine Fehlfunktion des Katalysators vor, wenn die im Abgas vorhandenen Stickoxide gar nicht oder nicht mehr für die geltende Abgasnorm ausreichend über dem Katalysator reduziert werden. Im Falle einer Schwellwertüberschreitung, des vom Katalysatorhersteller festgelegten NO₂-Wertes, wird eine binäre Fehlermeldung an die OBD ausgegeben, die wiederum geeignete Maßnahmen ergreifen kann (Werkstattleuchte, Fehlermeldung, Eingriff in die Motorsteuerung usw.).

Das verwendete Katalysatormaterial wird in dem hier verfolgten Ansatz gleichzeitig auch als Selektivfilter für den NO₂-Sensor verwendet. Dabei dient die zweite Schicht des Katalysators nicht nur für die Katalyse der Reaktionen CO + O₂ → CO₂ und UHC + O₂ → CO₂ + H₂O, sondern bei Fehlfunktion des Katalysators auch zur Katalyse der Reaktion NO + O₂ → NO₂. So wird erreicht, dass im Normalfall nur O₂ und CO₂ die Sensoren erreichen, die kein Sensorsignal verursachen sollten. Bei Fehlfunktion kann NO bis in die Oxidationsschicht vordringen und wird dort zu NO₂ reagiert. Neben den Katalyseprodukt CO₂ und O₂ erreicht noch NO₂ die Sensoroberfläche und bewirkt so einen Anstieg des Widerstands. Die Leitfähigkeit wird umso geringer je größer die Fehlfunktion des Katalysators ist. Gleiches gilt auch für die fortschreitende Alterung des Katalysators, da hier die Fläche mit höherem NO₂-Anteil über die Sensorelektrode wächst.

### Direkte Steuerung des katalytischen Reduktionsprozesses

Neben der einfachen Fehlfunktionsüberwachung ist auch eine direkt Steuerung des Katalysators durch den Sensor denkbar. In den aktuellen SCR-Katalysatoren werden die Reduktionsmittel (z.B. Harnstoff) kontinuierlich mit dem Abgas über den Katalysator zugeführt. In dem Fall, dass das Fahrzeug nicht in Betrieb ist, kann der nichtreagierte Harnstoff in Düsen und sonstigen Abgasleitungen einfrieren. Mit bauteilintegrierten NOₓ-, HC- und Ammoniak-Sensoren ist es möglich, die Reduktionsmittel nur dann in das System zu führen wenn es benötigt ist.

### Steuerung und Kontrolle von Verbrennungssystemen:

Kombiniert mit katalytischen Verbrennungskatalysatoren können integrierten Gassensoren die Verbrennungsprozesse der über Aktuatoren eingeführten Luft/Brennstoff-Steuerung kontrollieren, optimieren und protokollieren.

Systemintegrierte Gassensoren ermöglichen die Verbrennungssteuerung in mobilen Systemen.

### Bei Brennstoffzellen H₂-Reformerkatalysator und H₂-Detektor:

Die Funktion der Rh-basierenden H₂-reformerschichten von Brennstoffzellen werden durch integrierte H₂-Sensoren beobachtet und gleichzeitig die Konzentration und Reinheit von H₂ bestimmt.

## Patentansprüche

1. Vorrichtung zur qualitativen und/oder quantitativen Bestimmung wenigstens einer Komponente eines chemisch reduzierbaren Gasgemisches umfassend
a) eine Reduktionsschicht zur chemischen Reduktion wenigstens einer Komponente des Gasgemisches,
b) eine Oxidationsschicht zur teilweisen oder vollständigen Rückoxidation der durch die Reduktionsschicht chemisch reduzierten Komponente/n und
c) eine selektive Gassensor-Elektrode aus einem einfachen, einem binären oder einem dotierten Übergangsmetalloxid oder einem Halbleiteroxid zur qualitativen und/oder quantitativen Bestimmung wenigstens einer der rückoxidierten Komponenten, wobei die zu reduzierende Komponente auf ihrem Weg aus dem Gasgemisch zur Gassensor-Elektrode zunächst die Reduktionsschicht und dann die Oxidationsschicht passiert.

2. Vorrichtung nach Anspruch 1, **dadurch gekennzeichnet, dass** die Gassensor-Elektrode oder/und die Reduktionsschicht oder/und die Oxidationsschicht mit einem Trägersubstrat verbunden ist/sind.

3. Vorrichtung nach Anspruch 2, **dadurch gekennzeichnet, dass** das Trägersubstrat ein keramischer Waben körper oder eine Metallfolie umfasst, wobei sich zwischen der Metallfolie und der Gassensor-Elektrode eine elektrisch nicht leitende Schicht befindet.

4. Vorrichtung nach einem der Ansprüche 1 bis 3, **dadurch gekennzeichnet, dass** die Reduktions- und die Oxidationsschicht gasdurchlässige Schichten mindestens eines komplexen Oxids mit einer Spinell-, Perovskit- oder Aluminat-Struktur und mindestens ein Edelmetall umfassen.

5. Vorrichtung nach einem der Ansprüche 1 bis 4, **dadurch gekennzeichnet, dass** die Reduktionsschicht Pd und/oder Rh und/oder die Oxidationsschicht Pt enthält/enthalten.

6. Vorrichtung nach einem der Ansprüche 1 bis 5, **dadurch gekennzeichnet, dass** mindestens zwei Gassensor-Elektroden in Bezug auf eine Strömungsrichtung des Gasgemisches räumlich hintereinander angeordnet sind.

7. Abgaskatalysator oder Reformerkatalysator, insbesondere ein Drei-Wege-Katalysator, ein Selective Catalytic Reduction(SCR)-Katalysator oder ein NOₓ-Speicherkatalysator, umfassend eine Vorrichtung nach einem der Ansprüche 1 bis 6.

8. Verfahren zur Herstellung einer Vorrichtung nach einem der Ansprüche 1 bis 6, **dadurch gekennzeichnet, dass** man eine gegebenenfalls auf einem Trägersubstrat befindliche Gassensor-Elcktrode gemäss Ansprüche 1 bis 6 vorlegt und diese mit einer Oxidations- und einer Reduktionsschicht gemäss Ansprüche 1 bis 6 versieht,

9. Verfahren nach Anspruch 8, **dadurch gekennzeichnet, dass** man die Gassensor-Elektrode durch CVD-, PVD-, Sol-Gel-, Plasmaspritz-, Siebdruck-, PLD-, Plattier- oder Tauchverfahren erzeugt.

10. Verfahren nach einem der Ansprüche 8 oder 9, **dadurch gekennzeichnet, dass** man die Oxidations- und/oder Reduktionsschicht durch ein Sol-/Gel-Verfahren oder Tauchverfahren herstellt.

11. Verfahren zur Überwachung der NOₓ-Emissionen eines Fahrzeuges mit einem Abgaskatalysator oder Reformerkatalysator nach Anspruch 7, **dadurch gekennzeichnet, dass** man das Luft-Treibstoffverhältnis in Abhängigkeit von der/den Menge/n der Komponente/n variiert, für die die Gassensor-Elektrode selektiv ist.

12. Verwendung einer Vorrichtung nach einem der Ansprüche 1 bis 6 zur qualitativen und/oder quantitativen Bestimmung wenigstens einer Komponente eines chemisch reduzierbaren Gasgemisches.

13. Verwendung nach Anspruch 12, **dadurch gekennzeichnet, dass** das Gasgemisch ein Abgas eines Verbrennungsmotors ist.

## Claims

1. A device for the qualitative and/or quantitative determination of at least one component of a chemically reducible gas mixture, comprising:
a) a reduction layer for the chemical reduction of at least one component of the gas mixture;
b) an oxidation layer for the partial or complete back oxidation of the component(s) chemically reduced by the reduction layer; and
c) a selective gas sensor electrode made of a simple or binary or doped transition metal oxide or semiconductor oxide for the qualitative and/or quantitative determination of at least one of the back-oxidized components;
wherein the component to be reduced passes first the reduction layer and then the oxidation layer on its way from the gas mixture to the gas sensor electrode.

2. The device according to claim 1, **characterized in that** the gas sensor electrode or/and the reduction layer or/and the oxidation layer are bonded to a support substrate.

3. The device according to claim 2, **characterized in that** the support substrate includes a ceramic honeycomb structure or a metal foil, wherein an electrically non-conductive layer is provided between the metal foil and the gas sensor electrode.

4. The device according to any of claims 1 to 3, **characterized in that** the reduction and oxidation layers include gas-permeable layers of at least one complex oxide having a spinel, perovskite or aluminate structure and at least one precious metal.

5. The device according to any of claims 1 to 4, **characterized in that** the reduction layer contains Pd and/or Rh, and/or the oxidation layer contains Pt.

6. The device according to any of claims 1 to 5, **characterized in that** at least two gas sensor electrodes are arranged spatially one behind the other with respect to a flow direction of the gas mixture.

7. An exhaust gas catalytic converter or reformer converter, specially a three-way catalytic converter, a selective catalytic reduction (SCR) converter, or an NOₓ storage catalytic converter, comprising a device according to any of claims 1 to 6.

8. A process for preparing a device according to any of claims 1 to 6, **characterized in that** a gas sensor electrode according to claims 1 to 6, optionally supported on a support substrate, is provided and coated with oxidation and reduction layers according to claims 1 to 6.

9. The process according to claim 8, **characterized in that** the gas sensor electrode is produced by CVD, PVD, sol-gel, plasma spraying, screen printing, PLD, plating or immersion processes.

10. The process according to either of claims 8 or 9, **characterized in that** said oxidation and/or reduction layer is prepared by a sol-gel process or an immersion process.

11. A process for monitoring the NOₓ emissions of a vehicle having an exhaust gas catalytic converter or reformer converter according to claim 7, **characterized in that** the air-to-fuel ratio is varied as a function of the quantity or quantities of the component(s) for which the gas sensor electrode is selective.

12. Use of a device according to any of claims 1 to 6 for the qualitative and/or quantitative determination of at least one component of a chemically reducible gas mixture.

13. The use according to claim 12, **characterized in that** the gas mixture is an exhaust gas of an internal combustion engine.

## Revendications

1. Dispositif pour la détermination qualitative et/ou quantitative d'au moins un composant d'un mélange de gaz chimiquement réductible, comprenant
a) une couche réductrice pour la réduction chimique d'au moins un composant dudit mélange de gaz ;
b) une couche oxydante pour la réoxydation partielle ou complète dudit ou desdits composant/s chimiquement réduits par la couche réductrice ; et
c) une électrode de détection de gaz sélective en un oxyde de métal de transition simple, binaire ou dopé ou en un oxyde semi-conducteur pour la détermination qualitative et/ou quantitative d'au moins un des composants réoxydés ;
dans lequel le composant à réduire passe d'abord la couche réductrice et ensuite la couche oxydante sur son chemin du mélange de gaz à l'électrode de détection de gaz.

2. Dispositif selon la revendication 1, **caractérisé en ce que** ladite électrode de détection de gaz ou/et ladite couche réductrice ou/et ladite couche oxydante est liée/sont liées à un substrat de support.

3. Dispositif selon la revendication 2, **caractérisé en ce que** ledit substrat de support comprend une structure en nid d'abeille céramique ou une feuille métallique, une couche électriquement non conductrice étant procurée entre ladite feuille métallique et ladite électrode de détection de gaz.

4. Dispositif selon l'une quelconque des revendications 1 à 3, **caractérisé en ce que** lesdites couches réductrice et oxydante comprennent des couches perméables aux gaz en au moins un oxyde complexe ayant une structure de spinelle, de pérovskite ou d'aluminate, et au moins un métal précieux.

5. Dispositif selon l'une quelconque des revendications 1 à 4, **caractérisé en ce que** ladite couche réductrice contient du Pd et/ou du Rh, et/ou ladite couche oxydante contient du Pt.

6. Dispositif selon l'une quelconque des revendications 1 à 5, **caractérisé en ce qu'**au moins deux électrodes de détection de gaz sont spatialement disposées l'une derrière l'autre dans la direction d'écoulement du mélange de gaz.

7. Catalyseur de gaz d'échappement ou catalyseur de reformage, notamment un catalyseur à trois voies, un catalyseur à réduction catalytique sélective (SCR) ou un catalyseur à accumulation NOₓ, comprenant un dispositif selon l'une quelconque des revendications 1 à 6.

8. Procédé pour produire un dispositif selon l'une quelconque des revendications 1 à 6, **caractérisé en ce que** l'on procure une électrode de détection de gaz selon les revendications 1 à 6, éventuellement supportée sur un substrat de support, et l'on applique une couche oxydante et une couche réductrice selon les revendications 1 à 6 dessus.

9. Procédé selon la revendication 8, **caractérisé en ce que** ladite électrode de détection de gaz est générée par un procédé CVD, PVD, sol-gel, de pulvérisation au plasma, de sérigraphie, PLD, de placage ou d'immersion.

10. Procédé selon l'une des revendications 8 ou 9, **caractérisé en ce que** ladite couche oxydante et/ou couche réductrice est préparée par un procédé sol-gel ou d'immersion.

11. Procédé pour surveiller les émissions de NOₓ d'une véhicule ayant un catalyseur de gaz d'échappement ou un catalyseur de reformage selon la revendication 7, **caractérisé en ce que** le rapport air/carburant est varié en fonction des quantités des composants pour lesquels ladite électrode de détection de gaz est sélective.

12. Utilisation d'un dispositif selon l'une quelconque des revendications 1 à 6 pour la détermination qualitative et/ou quantitative d'au moins un composant d'un mélange de gaz chimiquement réductible.

13. Utilisation selon la revendication 12, **caractérisée en ce que** ledit mélange de gaz est un gaz d'échappement d'un moteur à combustion interne.
